Europäisches Patentamt

European Patent Office

Office européen des brevets

(11) Veröffentlichungsnummer: **0 563 454 A1**

## (12) EUROPÄISCHE PATENTANMELDUNG

(21) Anmeldenummer: **92122169.3**

(22) Anmeldetag: **30.12.92**

(51) Int. Cl.5: **A61B 3/10**, A61B 3/103,
A61B 3/107, G01B 11/24

(30) Priorität: **30.03.92 DE 4210384**

(43) Veröffentlichungstag der Anmeldung:
**06.10.93 Patentblatt 93/40**

(84) Benannte Vertragsstaaten:
**CH DE FR GB IT LI**

(71) Anmelder: **Stiller, Henning, Dipl.-Phys.**
**Alardusstrasse 9**
**D-20255 Hamburg(DE)**

(72) Erfinder: **Stiller, Henning, Dipl.-Phys.**
**Alardusstrasse 9**
**W-2000 Hamburg 20(DE)**
Erfinder: **Oechsner, Ulrich, Dipl.-Phys.**
**Hegholt 34e**
**W-2000 Hamburg 71(DE)**
Erfinder: **Rassow, Bernhard, Prof. Dr.rer.nat.**
**Heinsonweg 38c**
**W-2000 Hamburg 67(DE)**

(74) Vertreter: **Glawe, Delfs, Moll & Partner**
**Patentanwälte,**
**Postfach 26 01 62**
**D-80058 München (DE)**

(54) **Verfahren und Vorrichtung zum Untersuchen des Auges.**

(57) Bei dem Verfahren und der Vorrichtung zum Untersuchen des Auges mit einer Lichtquelle und einem Interferometer wird das Auge in einem ersten Strahlengang angeordnet und die von einer Grenzfläche des Auges zurückgeworfene Objektlichtwelle mit einer Referenzlichtwelle zur Interferenz gebracht. Die Interferenzerscheinung wird detektiert und ausgewertet, wobei erfindungsgemäß vorgesehen ist, daß die Auswertung mittels mindestens eines optoelektronischen Sensors erfolgt und die Referenzlichtwelle durch definierte optische Elemente zur Interferenz mit der Objektlichtwelle geführt wird.

Figur 1

EP 0 563 454 A1

Die Erfindung betrifft ein Verfahren zum Untersuchen des Auges mit einer Lichtquelle und einem Interferometer, bei dem in einem ersten Strahlengang das Auge angeordnet wird und bei dem die von einer Grenzfläche des Auges zurückgeworfene Objektlichtwelle mit einer Referenzlichtwelle zur Interferenz gebracht wird. Die Erfindung betrifft weiterhin eine Vorrichtung zur Durchführung des Verfahrens.

Verfahren und Vorrichtungen zum Untersuchen des Auges werden für die Bestimmung der optischen und der anatomischen Eigen schaften des Auges verwendet. Dazu zählen die Refraktometrie, die Topographie der Hornhaut und die Tomographie des Auges. Die Vorrichtungen können dabei nicht nur von Augenärzten verwendet werden, sondern auch von Optikern. Sie können auch zu lediglich reinen Diagnosezwecken verwendet werden, bei denen nur Feststellungen über die Refraktion, die Hornhautkrümmung oder beispielsweise die Fundustopographie getroffen werden sollen, ohne daß dabei eine therapeutische Maßnahme zur Korrektur einer eventuell festgestellten Anormalität vorgesehen ist.

Unter einem Augen-Refraktometer versteht man eine Apparatur, die die durch die Augenoptik zur Retina optisch konjugierte Ebene (den Fernpunkt) auffindet, d.h. die Fehl- oder Rechtsichtigkeit bestimmt.

Die besondere Problematik der Refraktionsbestimmung des Auges liegt darin, daß der hintere Halbraum des optischen Systems, nämlich das Augeninnere, einer direkten Messung, z.B. durch einen Detektor, nicht zugänglich ist. Augenrefraktionsbestim mungen werden z.Zt. mit folgenden vier verschiedenen Meßprin zipien realisiert: Skiaskopie-, Scheiner-, Bildschärfe- und Schneidenverfahren (Rassow, B./Wesemann, W., Ophthalmologischoptische Instrumente, Stuttgart: Enke-Verlag 1987). Bei allen Verfahren wird zunächst der Augenhintergrund beleuchtet. Das von dort reflektierte Licht wird zum Auffinden der Fernpunkte verwendet. Im Allgemeinen bildet das Auge um die optische Achse herum nicht kugelsymmetrisch ab, sondern das optische Augensystem hat eine zylindrische Komponente. Daher muß die Lage der Fernpunkte in verschiedenen Meridianen bestimmt werden.

Unter Topographie der Hornhaut versteht man ein Verfahren, das die Oberflächengestalt der Hornhaut vermißt. Da auch die Horn haut im allgemeinen nicht rotationssymmetrisch ist, besteht ebenfalls das Problem, daß in mehren Meridianen gemessen werden muß.

Bei der Tomographie des Auges handelt es sich um ein bildgebendes Verfahren (wie beispielsweise die Ultraschalltechnik), mit dem die anatomischen Strukturen des Auges dargestellt werden.

Durch eine solche Darstellung kann beispielsweise die Bulbuslänge (Abstand Hornhaut - Augenhintergrund) oder die Dicke der Hornhaut, aber auch die Topographie des Fundus und dessen Schichten bestimmt werden.

Mit einer vorbekannten Vorrichtung und einem vorbekannten Verfahren der eingangs genannten Art (DE 32 01 801 A1) können Teilstrecken des lebenden Auges gemessen werden, z.B. die optische Länge der axialen Teilstrecke von der Hornhautvorderfläche zur Retina. Dies geschieht dadurch, daß von der Hornhautvorderfläche und von der Retina zurückgeworfenes Licht miteinander zur Interferenz gebracht werden. Wird eine Lichtquelle kurzer Kohärenzlänge benutzt, so ist Interferenz nur dann beobachtbar, wenn der Wegunterschied zwischen der Hornhautvorderfläche und der Retina durch eine optische Verzögerung bis auf die Kohärenzlänge kompensiert wird. Durch Messen der Kompenstionslänge kann die optische Länge der fraglichen Teilstrecke bestimmt werden. Der Nachteil dieses Verfahrens und dieser Vorrichtung ist, daß nur Information über die Länge von Teilstrecken des Auges zu erhalten ist, was selbstverständlich nur ein ganz geringer Teil der Information ist, die man für eine umfangreiche Diagnostik der optischen und anatomischen Eigenschaften des Auges benötigt.

Die Aufgabe der Erfindung besteht in der Schaffung einer Vor richtung und eines Verfahrens der eingangs genannten Art, mit denen wesentlich umfangreichere Informationen über das Auge gewonnen werden können.

Die erfindungsgemäße Lösung besteht einerseits in einem Verfahren, daß sich dadurch auszeichnet, daß die Detektion der Interferenz mittels mindestens eines opto-elektronischen Sensors erfolgt und die Referenzlichtwelle durch definierte optische Elemente zur Interferenz mit der Objektlichtwelle geführt wird.

Die optischen Elemente (z.B. Spiegel und Linsen) sind dadurch definert, daß sie entweder Teile der Apparatur darstellen und dadurch eine wohlbekannte Referenzlichtwelle erzeugen, bezüglich der die Objektlichtwelle und damit die gewünschte Meßgröße mit Hilfe der Interferenzerscheinung bestimmt wird. Wird z.B. das aus der Rückseite einer Laserdiode austretende Licht ohne weitere Führung durch brechende oder spiegelnde Elemente mit der Objektlichtwelle zur Überlagerung gebracht, so ist in diesem Sinne die Austrittsöffnung der Lichtquelle als definiertes optisches Element zu verstehen. Es kann aber auch, wie im Falle der Tomographie, wünschenswert sein, das Auge bezüglich einer seiner Grenzflächen, z.B. der Kornhaut zu vermessen, weil dann axiale Augenbewegungen während der Messung keinen Fehler produzieren. Dazu muß die Referenzlichtwelle an der Grenzfläche reflektiert werden, bezüglich der vermessen werden soll und es muß die Topographie dieser Grenzfläche bekannt sein. Im Gegensatz zur vorbekannten Vorrichtung erlaubt die vorliegende Erfindung die Bestimmung der Topographie der Hornhaut, so daß die Hornhaut als definiertes optische Element zur Führung der Referenzlichtwelle mit einbezogen werden kann. Es kann erfindungsgemäß nicht nur eine axiale Distanz wie bei der vorbekannten Vorrichtung bestimmt werden. Die Detektion erfolgt auch nicht wie bei der vorbekannten Vorrichtung durch visuelle Beobachtung, sondern mittels eines opto-elektronischen Sensors, was nicht nur die automatische Durchführung von Messungen erleichtert, sondern auch mit Hilfe geeigneter Rechenverfahren erlaubt, genauere Informationen über das Auge, z.B. die Topographie der Hornhaut oder die Tomographie des Auges, zu erhalten.

Eine Vorrichtung zum Untersuchen des Auges mit einer Lichtquelle und einem Interferometer mit einem Interferometerarm, in dessen Strahlengang das zu untersuchende Auge bringbar ist, mit Einrichtungen zur Detektion und Auswertung der Interferenz zwischen der von einer Grenzfläche des Auges zurückgeworfene Objektlichtwelle mit einer Referenzlichtwelle, zeichnet sich erfindungsgemäß dadurch aus, daß die Detektionseinrichtungen mindestens einen opto-elektronischen Sensor aufweisen und die Referenzlichtwelle durch definierte optische Elemente zur Interferenz mit der Objektlichtwelle geführt wird.

Zur Refraktometrie wird eine Referenz- und eine Objektlichtwelle benötigt, die zueinander kohärent sind. Diese beiden Wellen können z.B. dadurch entstehen, daß aus verschiedenen Öffnungen einer oder mehrerer Lichtquellen phasengekoppeltes Licht austritt. Die Referenzlichtwelle kann z.B. aus der Rückseite einer Laserdiode austreten und direkt auf den Sensor geführt werden. Referenz- und Objektlichtwelle können aber auch durch Strahl- oder Energieteilung der Strahlung einer Lichtquelle erzeugt werden, z.B. mittels separater Interferometerarme (Michelson-Interferometer) oder mittels einer teilreflektierenden Fläche in einem Arm des Interferometers (Fizeau-Interferometer). Die Objektlichtwelle wird in das Auge gelenkt, vom Augen hintergrund reflektiert und von der Augenoptik und ggfs. optischen Bauteilen auf den Sensor gelenkt, wo sie mit der von definierten optischen Elementen ebenfalls auf den Sensor geführten Referenzlichtwelle zur Überlagerung kommt. Durch ein Polarisationsfilter kann unerwünschtes Streulicht, das nicht zum Kontrast des Interferenzbildes beiträgt (z.B. ein in der Polarisationsrich tung veränderter Anteil) abgeschwächt werden. Mit dieser Vorrichtung werden Interferogramme erzeugt, die in der Sensorebene erfaßt werden und redundant die gesamte Information über die Refraktion des untersuchten Auges enthalten.

Da die Wellenfront der Referenzlichtwelle bekannt ist, kann nämlich aus der Interferenzfigur die Objektlichtwelle bestimmt werden, d.h. es läßt sich die Lage des Fernpunktes des Auges bestimmen. Hat das optische System des Auges eine zylindrische Komponente, so erhält man für unterschiedliche Meridiane unterschiedliche Lagen der Fernpunkte. Anders ausgedrückt, sowohl die Referenz- als auch die Objektwelle treffen auf die Detektorebene und interferieren. Das Interferenzmuster hängt vom Abstand der beiden virtuellen Lichtquellen dieser Strahlenbündel ab und enthält insofern die gewünschte Information über die Lage der Fernpunkte des Auges.

Zweckmäßigerweise ist in der Detektorebene eine CCD-Matrix angeordnet, so daß die Daten des Interferogramms in einen Rechner gegeben und dort aufbereitet werden können.

In der Ophthalmologie beschreibt man Abweichungen von der Rechtsichtigkeit (Ametropie) angenähert durch die Brechkraft einer Kombination aus einer sphärischen und einer zylindrischen Linse. Ein solches optisches System ist vollständig durch ein Parametertripel festgelegt: die sphärische und die zylindrische Brechkraft und die Orientierung der Achse der Zylinderlinse. Jedem Parametertripel entspricht genau ein Interferogramm auf der CCD-Matrix. Durch Variationen dieses Tripels läßt sich dasjenige Interferogramm bestimmen, das die mit der CCD-Matrix gemessene Intensitätsverteilung mit dem kleinsten Fehler annähert. Dieses Tripel ist die gemessene Ametropie. Sie soll aus den Daten des Interferogramms berechnet werden. Das Verfahren ist aber nicht beschränkt auf die Messung von sphärischen und zylindrischen Refraktionsfehlern des Auges, sondern kann prinzipiell jede beliebige Form von Fehlsichtigkeit bestimmen.

Bei einer Relativbewegung zwischen Meßapparatur und Objekt (Patientenauge) können die Meßergebnisse verfälscht und der Kontrast des Interferogramms vermindert werden. Aus diesem Grunde ist es zweckmäßig, die Messung in einem Zeitraum durchzuführen, in dem verglichen mit der Wellenlänge des Lichtes nur eine kleine Abstandsänderung zwischen Apparatur und Patient bzw. Auge erfolgt. Dieses Problem kann durch einen kurzen Lichtpuls der Laserdiode teilweise gelöst werden. Da während eines Pulses jedoch eine Wellenlängenänderung des Lichtes auftritt und diese ebenfalls zu einer Kontrastverminderung des Interferenzbildes führt, wird die Detektion der CCD-Kamera während des ersten Teils des Pulses, in dem die Wellenlängen änderung besonders stark ist, verhindert, z.B. indem sie lichtunempfindlich geschaltet wird.

Die Messungen werden zweckmäßigerweise bei entspannter Akkommodation des Auges durchgeführt. Um dem Patienten diese Akkommodationsentspannung zu erleichtern, kann zweckmäßiger weise vorgesehen werden, daß in einem optischen Arm der Vorrichtung eine Testmarke eingespiegelt wird, deren scheinbare Entfernung vom Auge durch Verschieben einer Spiegelfläche veränderbar ist. Der Patient oder die sonstige zu untersuchende Person versucht, die Testmarke scharf zu sehen. Wird die Testmarke in die Fernebene des Auges gelegt, so kann die Akkommodation entspannt werden.

Es ist zweckmäßig, die kohärente Lichtquelle als Punktlicht quelle optisch an den gleichen Ort wie die Testmarke zu positionieren, d.h. ihre scheinbare Entfernung zusammen mit der Testmarke zu verändern. Dann wird nämlich, wenn sich die Testmarke in der Fernebene des Auges befindet und damit die Akkommodation entspannt wird, die Meßlichtquelle scharf auf den Augenhintergrund abgebildet. Das hat zur Folge, daß das in der Detektorebene entstehende Interferenzbild am wenigsten durch Speckle gestört ist und damit am besten für die Auswertung geeignet ist.

Die Position der Testmarke, für die die Akkommodation entspannt wird, sich Testmarke und Punktlichtquelle also in der Fernebene des Auges befinden, läßt sich automatisch detektieren. Wird nämlich Objekt- und Referenzlichtwelle beispielsweise durch einen Strahlteiler auf eine Photodiode gelenkt und ist die Frequenz der beiden Wellen unterschiedlich, so detektiert die Photodiode die resultierende Schwebungsfrequenz des Inter ferenzbildes dann, wenn die mittlere Specklegröße etwa gleich groß oder größer als die Detektorfläche ist, der Sensor also nicht über mehrere Speckle mittelt. Die mittlere Specklegröße ist aber umso größer, je näher sich die kohärente Punktlichtquelle und damit auch die Testmarke an der Fernebene des Auges befindet. Die Schwebungsfrequenz im Photodiodensig nal eignet sich also als Meßsignal zum Auffinden der Fernebene und damit der Akkommodationsentspannung des Auges. Außerdem kann die so gewonnene Information über die Lage der Fernebene als Vorabinformation für die Auswertung des Interferogramms dienen, aus dem dann die Refraktion präzise bestimmt wird. Die für die Schwebung notwendige Frequenzdifferenz zwischen Objekt- und Referenzlichtwelle kann beispielsweise durch eine Dopplerverschiebung der Referenzlichtwelle erreicht werden.

Zweckmäßigerweise ist der Laser ein Infrarotlaser, da dann die untersuchte Person überhaupt nichts von dem untersuchenden Laserstrahl bemerkt. Es ist besonders zweckmäßig, wenn der Laser eine Laserdiode ist.

Die Vorrichtung der Erfindung hat den Vorteil, daß sie automatisch betrieben werden kann.

Die Vorrichtung der Erfindung kann auch zur Topographie der Hornhaut (Keratometrie) verwendet werden. Bei der erfindungsgemäßen Lösung zur Keratometrie wird die Objektlichtwelle durch ein vor das Auge eingeschwenktes Objektiv etwa auf den Mittelpunkt der durch die Hornhautkrümmung gebildeten Kugel fokussiert. Das von der Hornhaut reflektierte Licht wird durch das Objektiv und ggfs. weitere optische Bauteile auf die Detektorebene geführt, wo es mit der Referenzlichtwelle interferiert. Als Referenzlichtwelle kann z.B. ein in einem separaten Interferometerarm von einem Spiegel reflektierter Lichtstrahl oder der Reflex von einer Linsenoberfläche des Objektivs dienen.

Da die Wellenfront der Referenzlichtwelle bekannt ist, kann aus der Interferenzfigur die Objektlichtwelle bestimmt werden. Bei bekannten Abbildungseigenschaften der optischen Bauteile und des Objektivs läßt sich daraus die Wellenfront nach der Reflektion am Auge rekonstruieren. Ist die Wellenfront der auf das Auge einfallenden Welle und die Position des Auges relativ zur Meßapparatur bekannt, so lässt sich daraus die Topograhie des Reflektors, also der Hornhaut bestimmen.

Zweckmäßigerweise kommt ein Objektiv mit großer Apertur zur Anwendung, so daß von einer möglichst großen Fläche der Hornhaut reflektiertes Licht auf den Detektor abgebildet und damit eine möglichst große vermeßbare Fläche der Hornhaut erreicht werden kann.

Ebenfalls im Interesse einer möglichst großen vermeßbaren Fläche der Hornhaut ist der Strahlengang zweckmäßigerweise so konzipiert, daß der Fokus mit dem Mittelpunkt der von der Hornhaut näherungsweise gebildeten Kugel zusammenfällt. Dann werden nämlich im Idealfall einer kugelförmigen Hornhaut alle Strahlen in sich selbst zurückreflektiert, so daß sowohl für die einfallende als auch für die reflektierte Welle die gesamte Apertur des Objektivs genutzt werden kann und damit die Größe des vermeßbaren Hornhautareals optimiert ist. Die Fokussierung auf den Kugelmittelpunkt hat den weiteren Vorteil, daß unabhängig von der Krümmung der Hornhaut immer etwa gleich viele Interferenzringe auf dem CCD-Detektor entstehen. Andernfalls kann es bei extremen Hornhautkrümmungen zu einer sehr großen Anzahl von Interferenzringen kommen, die dann möglicherweise vom Detektor nicht mehr aufgelöst werden kann.

Eine zu große Anzahl von Interferenzringen und damit ein zu feines, vom Detektor nicht mehr auflösbares Interferenzmuster kann auch durch die Verwendung von Licht mit zwei verschiedenen Wellenlängen $\lambda_1$ und $\lambda_2$ (z.B. durch Einkopplung zweier Laserdioden) vermieden werden. Dann entsteht nämlich ein Interferenzmuster dem eine gröberes Muster (eine Schwebung) überlagert ist, das einer sogenannten synthetischen Wellenlänge von

$\Lambda = \lambda_1\lambda_2/(\lambda_1-\lambda_2)$ entspricht.

Um sicher zu stellen, daß bei unterschiedlichen Hornhautkrümmungen das vermeßbare Hornhautareal immer etwa gleich groß ist, ist es zweckmäßig, die Anordnung von Linsen und CCD-Detektor im Detektorarm so zu wählen, daß Auge und Detektor zueinander optisch konjugiert sind. Dies hat den weiteren Vorteil, daß der CCD-Detektor ein Bild des Auges liefert und damit auch zur Justierung der Meßapparatur auf das Auge verwendet werden kann.

Die für die Fokussierung auf den Kugelmittelpunkt notwendige Justierung der Meßapparatur relativ zum Patientenauge kann zweckmäßigerweise mit Hilfe einer Photo- oder Quadrantendiode erfolgen, die so positioniert wird, daß sie im Falle in sich selbst zurückreflektierter Strahlen optisch konjugiert zur Punktlichtquelle ist. Das Signal der Diode eignet sich sowohl für einen automatischen Regelkreis als auch für die Justierung durch den Bediener. Statt dieser zusätzlichen Diode kann auch die Laserdiode selbst verwendet werden, da ihr Steuerstrom sehr empfindlich auf optische Rückkopplungen reagiert.

Ist der Abstand zwischen Meßapparatur und Patientenauge nicht bekannt, so lässt sich aus der geschilderten Messung nur die relative Topographie der Hornhaut bestimmen, also beispielsweise Abweichungen von der Kugelform, nicht jedoch der absolute Krümmungsradius. Der Abstand zwischen Meßapparatur und Patientenauge kann zweckmäßigerweise weißlichtinterferometrisch, also durch Licht mit kurzer Kohärenzzeit und hoher räumli cher Kohärenz bestimmt werden. Dazu kann die Laserdiode mit einer Schwenkvorrichtung gegen eine Lichtquelle mit den gewünschten Kohärenzeigenschaften ausgetauscht werden (z.B. eine Superlumineszenzdiode). In einer anderen zweckmäßigen Ausführungsform kann auf den Austausch der Laserdiode verzichtet werden. Sie emitiert nämlich das erforderliche kurzkohärente Licht, wenn ihrem Steuerstrom eine hochfrequente Modulation überlagert wird. Wird die Strahlung der Laserdiode in zwei zueinander verzögerte Wellen aufgeteilt (beispielsweise durch ein Michelson-Interferometer), so beobachtet man dann ein Interferenzbild in der Detektorebene, wenn die Laufzeit differenz zwischen Objekt- und Referenzlichtwelle bis auf die Kohärenzzeit gleich der Laufzeitdifferenz ist, die durch die zusätzlich eingeführte Verzögerung entsteht. Kennt man die eingeführte Laufzeitverzögerung und ist die Kohärenzlänge genügend kurz, so läßt sich mit Hilfe der Gruppengeschwindigkeit die Position des Reflektors der Objektwelle, also der Hornhaut, relativ zur Position des Reflektors der Referenzwelle, also der Apparatur, in der Größenordnung der Kohärenzlänge genau bestimmen.

Zur Einführung zweier relativ zueinander verzögerter Wellen der Laserdiode eignet sich ein Michelson-Interferometer auch deswegen besonders gut, weil bei gleicher Länge der beiden Interferometerarme ein Interferenzsignal beobachtbar ist und deshalb der Nullpunkt der Verzögerung leicht kalibriert werden kann.

Das Auftreten der Interferenzerscheinung kann in einer vor teilhaften Ausführungsform wie folgt automatisch detektiert werden: Die Länge der Verzögerungsstrecke wird zum Zwecke der Messung über den zu erwartenden Bereich mit gleichförmiger Geschwindigkeit variiert. Dadurch verschiebt sich wegen des Dopplereffekts die Frequenz der Referenzlichtwelle. Dies führt zu einer Schwebung (einem Pulsieren) der Interferenzringe, deren Frequenz gleich der Dopplerfrequenz ist. In der Detektorebene schwingt also die Intensität mit der Dopplerfrequenz, sofern das Interferenzbild auftritt, also die Laufzeitdifferenz zwischen Objekt- und Referenzstrahl bis auf die Kohärenzzeit gleich der eingeführten Laufzeitdifferenz ist. Das Auftreten dieser Intensitätsmodulation kann z.B. durch eine Photodiode detektiert und damit bei Kenntnis der zugehörigen Laufzeitverzögerung mit Hilfe der Gruppengeschwindigkeit der Abstand der Hornhaut des Patientenauges relativ zur Meßapparatur bestimmt werden.

Diese Meßgenauigkeit kann nach dem Noniusprinzip noch weiter erhöht werden, wenn das Interferenzbild zur Topographiebestimmung mit Zweiwellenlängeninterferometrie aufgenommen wird. Die Phase des niederfrequenten Schwebungsmusters enthält nämlich die Information über den Abstand der Hornhaut von der Apparatur mit sehr großer Präzision, allerdings nur modulo der Ortsperiode dieses Musters. Wird diese Mehrdeutigkeit durch die oben geschilderte Messung aufgehoben, dann lässt die zusätzliche Phaseninformation eine sehr genaue Bestimmung des gesuchten Abstandes zu.

Da für die Keratometrie eine Entspannung der Akkommodation nicht notwendig ist, dient die Testmarke, falls sie vorgesehen ist, in diesem Fall lediglich als Fixationshilfe für den Patienten zur richtigen Ausrichtung seines Auges entlang der optische Achse der Meßapparatur.

Die Vorrichung der Erfindung kann auch zur Tomographie des Auges verwendet werden. Für diese Messung wird Licht kurzer Kohärenzlänge benötigt, wie dies oben im Zusammenhang mit der Messung des Abstandes zwischen Meßapparatur und Patientenauge beschrieben wurde.

Wie weiter oben im Zusammenhang mit der Verwendung der Apparatur als Refraktometer beschrieben, beobachtet man in der Detektorebene ein Interferenzbild, das aus der Überlagerung von Referenzlichtwelle mit dem Reflex vom Augenhintergrund entsteht. Als Referenzlichtwelle kann für die Tomographie zweckmäßi gerweise auch der Reflex von der Hornhaut dienen, so daß relativ zur Hornhaut gemessen wird. Eine absolute Tomographie des Auges ergibt sich daraus mit Hilfe der oben geschilderten Topographie der Hornhaut. Die Verwendung des Hornhautreflexes als Referenzlichtwelle hat den Vorteil, daß axiale Augenbewegungen während der Messung nicht zu falschen Ergebnissen führen.

Um ein möglichst wenig durch Speckle gestörtes Interferenzbild zu erhalten ist es auch für die Tomographie zweckmäßig, wenn die Punktlichtquelle wie bei der Refraktometrie automatisch in die Fernebene des Auges gelegt wird. Wird kurzkohärentes Licht verwendet, so tritt eine Interferenzerscheinung durch die Überlagerung dieser beiden Reflexe nur dann auf, wenn deren Laufzeitdifferenz bis auf die Kohärenzzeit gleich einer wie oben im Zusammenhang mit dem Keratometer beschriebenen, zusätzlich eingeführten Laufzeitdifferenz ist. Kennt man die eingeführte Laufzeitverzögerung bei Auftreten des Interferenzbildes und ist die Kohärenzzeit genügend kurz, so läßt sich mit Hilfe der Gruppengeschwindigkeit die Position der beiden Reflektoren (in dem genannten Beispiel Hornhaut und Augenhintergrund) relativ zueinander in der Größenordnung der Kohärenzlänge genau bestimmen.

Auch für die Tomographie ist die Verwendung eines Michelson-Interferometers zur Verzögerung der beiden Wellen relativ zueinander zweckmäßig. Neben dem Vorteil der weiter oben geschilderten einfachen Kalibrierung werden nämlich im Michelson-Interferometer die beiden in der Detektorebene zur Überlagerung kommenden Wellen räumlich getrennt. Dadurch ist es möglich, ihre Intensität zum Beispiel durch Filter so anzupassen, daß die interferierenden Wellen in der Detektorebene gleiche Bestrahlungsstärke haben und sich somit der Kontrast des Interferenzbildes verbessert. Darüber hinaus kann in den beiden Interferometerarmen in einer zur Pupille des Auges optisch konjugierten Ebene durch Blenden eine Pupillenteilung für die beiden Wellen erreicht werden. In Kombination mit einer geeigneten Eintrittspupille der Apparatur kann so erreicht werden, daß nur die im Michelson-Interferometer verzögerte und an der Hornhaut reflektierte Referenzlichtwelle mit der unverzögerten und am Fundus reflektierten Objektlichtwelle in der Detektorebe ne überlagert werden. Dann verbessert sich der Kontrast des Interferenzbildes, weil inkohärente Reflexe ihn nicht herabsetzen. Als Blende kann auch eine geeignete Ausgestaltung der Spiegel des Michelson-Interferometers dienen.

Das Auftreten der Interferenzerscheinung kann in einer vor teilhaften Ausführungsform, wie oben im Zusammenhang mit der Verwendung der Apparatur als Keratometer beschrieben, automatisch detektiert werden. Beispielsweise können durch einen Strahlteiler in einem Arm die Reflexe auf eine Photodiode projiziert werden, die die mit der Interferenzerscheinung verbundene Intensitätsmodulation detektiert. Zur einfachen Justierung ist es vorteilhaft, wenn die Photodiode in einer Ebene liegt, die optisch zu einer 2 bis 3 cm vor dem Auge liegenden Ebene konjugiert ist. Dort ist nämlich der Strahldurchmesser groß genug, um auch bei leichter Dejustierung noch auf den Detektor zu fallen. Wird auch die Modulationstiefe des Signals (also der relative Kontrast des Interferenzbildes) aufgezeichnet, so läßt sich nicht nur eine Aussage über den Ort des Reflektors, sondern auch über dessen Reflektivität machen. Weil diese aber mit der räumlichen Struktur des Gewebes korreliert ist, erlaubt die beschriebene Meßmethode eine tomographische Darstellung des Augenhintergrundes entlang der Strahlrichtung. Wird die optische Achse des Apparatur um den Knotenpunkt des Auges gedreht, so läßt sich ein weiter Bereich des Augenhintergrundes tomographisch relativ zur Hornhaut darstellen. Wird wie oben beschrieben auch die Topographie der Hornhaut gemessen, so ist eine absolute tomographische Darstellung des Auges möglich.

Die Tomograhie beschränkt sich dabei nicht auf die Darstellung des Augenhintergrundes. Eine Interferenzerscheinung kann nämlich auch resultieren aus der Überlagerung des Hornhautreflexes mit Reflexen, die an anderen Grenzflächen im Auge (bspw. der Hornhautrückfläche oder den Linsenflächen) entstehen. Da es sich hierbei um spiegelnde Reflexe handelt und daher keine Speckle auftreten, ist es nicht notwendig die Punktlichtquelle auf die entsprechende Grenzfläche zu fokussieren. Vielmehr ist deren scheinbare Entfernung zur Erreichung eines guten Kontrastes des Interferenzbildes so einzustellen, daß der betrachtete Reflex und der Hornhautreflex möglichst wenig unterschiedliche Bestrahlungsstärke in der Detektorebene haben. Außerdem muß sie so gewählt sein, daß eine eine Ortsfrequenz des Interferenzbildes resultiert, die vom Detektor aufgelöst werden kann.

Das erfindungsgemäße Verfahren und die erfindungsgemäße Vorrichtung erlauben im Gegensatz zum Stand der Technik (DE-A-3 201 801) nicht nur die Vermessung von Teilstrecken, sondern eine tomographische Darstellung von weiten Bereichen des Auges, ähnlich den Ergebnissen, die mit Ultraschallverfahren erzielt werden können, im Vergleich dazu aber mit wesentlich besserer longitudinaler und lateraler Auflösung und ohne den für den Patienten unangenehmen physischen Kontakt mit dem Auge. Für solche tomographischen Messungen sind mehrere Voraussetzungen notwendig: 1.

Der relative Kontrast des Inter ferenzbildes ist eine Meßgröße und muß daher aufgezeichnet werden. 2. Um eine möglichst hohe Auflösung der relativen Kontrastmessung zu erreichen, muß der maximal mögliche Kontrast groß sein, d.h. der inkohärente Grundlichtanteil muß möglichst klein sein. 3. Die Topograhie der Hornhaut muß bekannt sein. 4. Soll die Messung muß auch bei fehlsichtigen Augen durchfürbar sein, so muß dies ohne Sehhilfe (Brille) möglich sein, da sonst die Strahlgeometrie und damit der vermessene Bereich des Auges unsicher sind und sich außerdem das Signal durch Reflektionen an der Brille verschlechtert. Alle genannten Anforderungen sind in der vorliegenden Erfindung im Gegensatz zur vorbekannten Vorrichtung und dem vorbekannten Verfahren erfüllt.

Die Erfindung wird im folgenden anhand von vorteilhaften Ausführungsformen unter Bezugnahme auf die beigefügten Zeichnungen beschrieben.

Es zeigen in schematischer Darstellung

Fig. 1: die Verwendung der erfindungsgemäßen Vorrichtung als Augenrefraktometer;

Fig. 2: die Verwendung der erfindungsgemäßen Vorrichtung für die Topographie der Hornhaut (Keratometer);

Fig. 3: die Verwendung der erfindungsgemäßen Vorrichtung für die Tomographie des Auges.

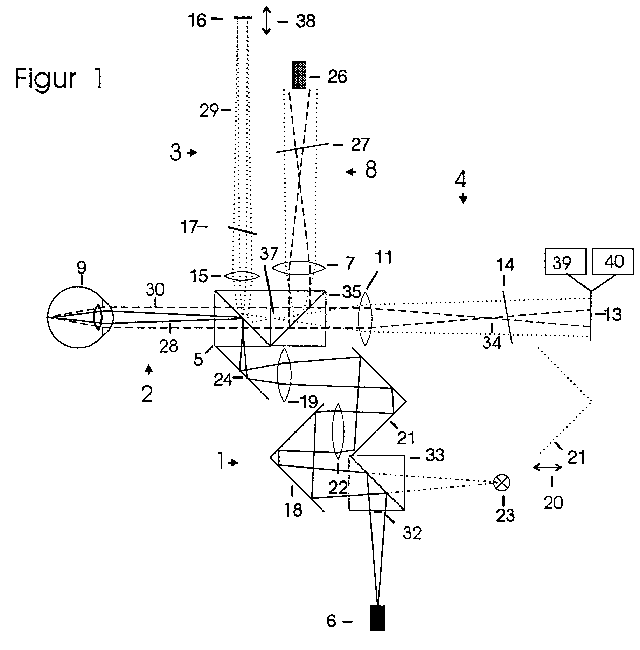

Die Vorrichtung der Erfindung der Fig. 1 besteht hauptsächlich aus vier optischen Armen 1, 2, 3 und 4, die senkrecht aufeinanderstehen und sich im Stahlteiler 5 treffen und einem weiteren optischen Arm 8, der durch den Strahlteiler 35 aus dem Arm 4 hervorgeht.

Am Ende des optischen Arms 1 befindet sich der Strahlenteiler 33, über den sowohl das divergente, mit durchgezogenen Strichen gezeichnete Licht der Laserdiode 6 als auch das mit Punkten und Strichen gezeichnete Licht von einem Fixationstarget 23 eingespiegelt werden kann. Nach Strahlumlenkung durch das Reflektionsprisma 18 bildet Linse 22 den Quellpunkt der Laserdiode bzw. das Fixationstarget in eine Bildebene ab, deren Abstand relativ zur Linse 19 durch das in Richtung des Doppelpfeils 20 verschiebare Reflektionprisma 21 variiert werden kann. Nach Abbildung durch Linse 19 und Reflektion am Spiegel 24 fällt das Licht auf den Strahlteiler 5 und wird dort in die ausgezogen gezeichneten Strahlen 28 und in die gepunktet gezeichnete Referenzstrahlen 29 aufgeteilt. Das Strahlenbündel 28 fällt in das Auge 9, so daß der Patient das Fixationstarget 23 erblickt. Er sieht ein scharfes Bild des Fixationstargets, wenn seine scheinbare Entfernung durch Variation der Lage des Prismas 21 in die Fernebene des Patientenauges gelegt wird. Weil die Akkommodation des Auges dem Bild des Fixationstargets zu folgen versucht, kann sie durch Vergrößern der scheinbaren Entfernung entspannt werden. Die Linse 19 hat dabei die Aufgabe, das Target so abzubilden, daß durch eine im Gerät durchführbare Lageveränderung des Prismas 21 sämtliche interessierende scheinbare Entfernungen eingestellt werden können. Es ist zweckmäßig die Brennweite der Linse 19 so zu wählen, daß ihr Brennpunkt mit dem Knotenpunkt des Auges 9 zusammenfällt; dann erscheint nämlich das Fixationstarget unabhängig von der scheinbaren Entfernung immer unter der gleichen Größe.

Gleichzeitig mit dem Fixationstarget wird auch das Licht der Laserdiode auf die Netzhaut fokussiert und von dort diffus reflektiert. Dieser Strahl tritt dann als gestreuter Strahl 30 aus dem Auge 9 aus; der austretende Strahl 30 ist gestrichelt dargestellt. Diese Objektlichtwelle 30 geht dann durch den Strahlteiler 5 hindurch und wird von einer Linse 11 im vierten optischen Arm 4 in den Bereich 34 fokussiert und auf einen Matrix-CCD-Sensor 13 projiziert. Die Objektlichtwelle 30 hat damit eine virtuelle Lichtquelle im Punkt 34.

Die Referenzlichtwelle 29 passiert eine Sammellinse 15 im dritten optischen Arm 3 und wird von einem Spiegel 16 reflektiert, wobei dann durch die Sammellinse 15 ein Fokussieren des Strahls auf einen Punkt 37 erfolgt. Mit Hilfe eines Abschwächers 17 kann die Intensität des Referenzstrahls 29 so eingestellt werden, daß er in der Detektorebene 13 ungefähr die Bestrahlungsstärke des Objektstrahles 30 hat. Das vom Vereinigungspunkt 37 des Referenzstrahles 29 ausgehende Licht wird von der Linse 11 auf die Matrix-CCD-Sensorenfläche 13 projiziert, wobei aus der Divergenz des Strahles eine virtuelle Lichtquelle für den Strahl 29 resultiert. Mit Hilfe des Strahlteilers 35 kann das Licht der Objekt- und Referenzlichtwellen auch in den optischen Arm 8 gelenkt werden, in dem es durch die Linse 7 auf die Photodiode 26 fällt.

In der Detektorebene erzeugt die diffus am Augenhintergrund gestreute Objektlichtwelle 30 ein Specklemuster. Die mittlere Specklegröße ist umso größer, je schärfer der Quellpunkt der Laserdiode auf den Augenhintergrund abgebildet wird, d.h. je näher das Bild des Quellpunktes der Laserdiode und damit auch des Fixationstargets an der Fernebene des Auges liegt. Wird der Spiegel 16 in Richtung des Doppelpfeils 38 bewegt, so entsteht daraus eine Dopplerverschiebung der Referenzlichtwelle. Die Überlagerung mit dem Objektlichtwelle führt dann zu einer Schwebung der Intensität, die von der Photodiode detektiert wird, sofern die mittlere Specklegröße etwa gleich groß oder größer als die Detektorfläche ist. Damit kann das Auftreten dieses Wechselsignals an der Photodiode 26 zur automatischen Detektion derjenigen Position des Prismas 21 dienen, für die die scheinbare Entfernung des Quellpunktes der Laserdiode und damit auch des Fixationstargets in der Fernebene des Auges liegt und die Akkommodation entspannt wird.

In der beschriebenen Situation der scharfen Abbildung des Quellpunktes der Laserdiode auf den Augenhintergrund ist die Specklegröße so groß, daß aus der Überlagerung von Objektlichtwelle 30 und Referenzlichtwelle 29 in der Detektorebene 13 ein durch Speckle wenig gestörtes Interferenzmuster entsteht, das die präzise Bestimmung der Lage des Punktes 34 in allen Meridianen, also die präzise Bestimmung der Refraktion des Auges 9 zuläßt. Zur Erhöhung des Kontrastes des Interferenzbildes kann unerwünschtes Streulicht mit Hilfe der Polarisationsfilter 14 bzw. 27 reduziert werden. Um eine Störung des Interferenzbildes durch den Reflex des Strahlenbündels 28 an der Hornhaut zu vermeiden, kann in einer durch die Linsen 19 und 22 zur Hornhaut optisch konjugierten Ebene eine Blende oder ein Filter einer solchen Größe eingefügt werden, daß der Reflex nicht auf die Detektoren 13 oder 26 fällt. Alternativ dazu kann die Kohärenzlänge des Lichts z.B. durch Pulsen der Laserdiode so verkürzt werden, daß durch den Hornhautreflex kein störendes Interferenzmuster ensteht.

Die Auswertung der Interferenzmuster auf der Fläche 13 der Matrix-CCD-Sensoren kann mit Hilfe eines Rechners 39 erfolgen. Bei der Vorrichtung der Erfindung kann das Auge mit Hilfe des Linsensystems 11 in die Detektorebene 13 abgebildet werden. Daher kann das auf dem CCD-Sensor entstehende Bild des Auges auf einem Bildschirm 40 angezeigt werden und damit zur Justierung der Apparatur dienen.

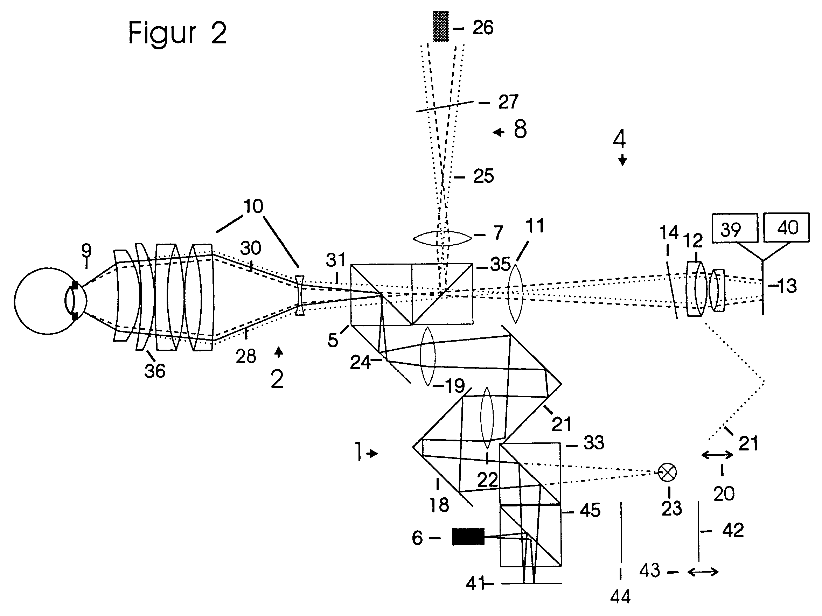

Wie für Fig. 1 geschildert, wird bei der Vorrichtung der Fig. 2 das Licht der Laserdiode 6 auf den Strahlteiler 5 geleitet, und von dort wird das ausgezogen gezeichnete Strahlenbündel 28 auf das Patientenauge 9 gelenkt. Die Stellung des Prismas 21 wird dabei so gewählt, daß das Strahlenbündel 28 in den Bereich des Mittelpunktes der durch die Hornhaut gebildeten Kugel fokussiert wird. Um einen möglichst großen Bereich der Hornhaut vermessen zu können, wird das Strahlenbündel 28 zunächst aufgeweitet und dann auf das Auge fokussiert; der an der Hornhaut reflektierte Strahl 30 ist gestrichelt dargestellt. Dieser Objektstrahl 30 passiert zunächst erneut das Objektiv 10, geht dann durch den Strahlteiler 5 und die Linse 11 hindurch und wird von dem Linsensystem 12 im vierten optischen Arm 4 auf einen Matrix-CCD-Sensor 13 projiziert.

Wie für Fig. 1 geschildert, kann der Arm 3 dazu dienen, eine Referenzlichtwelle zu erzeugen, die ebenfalls auf den CCD-Sensor 13 projiziert wird. In Figur 2 ist jedoch eine alternative Möglichkeit zur Erzeugung der Referenzlichtwelle dargestellt. Wird eine Linsenfläche des Objektivs 10 (bspw. eine Oberfäche 36 der Meniskuslinsen) nicht entspiegelt, so entsteht dort ein Reflex 31, der als Referenzlichtwelle dienen kann. Sie wird ähnlich der Objektlichtwelle 30 auf den Matrix-CCD-Sensor 13 geleitet. Dort entsteht dann ein Interferenzmuster, das in eindeutiger Weise die Information über die Wellenfront der Objektwelle und damit über die Form des Reflektors (der Hornhaut) enthält. Zur Erhöhung des Kontrastes des Interferenzbildes kann unerwünschtes Streulicht mit Hilfe der Polarisationsfilter 14 bzw. 27 reduziert werden. Zur Desensibilisierung der sehr empfindlichen interferometrischen Messung können zwei Interferenzmuster, die mit unterschiedlichen Wellenlängen 1 und 2 erzeugt werden, überlagert werden. Das kann z.B. realisiert werden, indem das Fixationstarget 23 gegen eine weitere Laserdiode mit der gewünschten Wellenlänge ausgetauscht wird.

Zur Ausrichtung des Auges entlang der optischen Achse der Apparatur kann wiederum das Fixationstarget 23 dienen. Weiter hin kann im optischen Arm 8, der durch den Strahlteiler 35 entsteht, im Bereich 25 eine Photo- oder Quadrantendiode eingeschwenkt werden, die so positioniert wird, daß sie für vom Objekt in sich selbst zurückreflektierte Strahlen im Fokus des Strahlenbündels 30 steht. Sie kann daher über einen Regelkreis zur automatischen Einjustierung der Fokussierung des Strahls 28 auf den Mittelpunkt der durch die Hornhaut gebildeten Kugel dienen. Um den für die absolute Topographiebestimmung notwendigen Abstand der Hornhaut von der Apparatur messen zu können, kann das Licht der Laserdiode 6 vor der Einspiegelung durch den Strahlteiler 33 in den optischen Arm 1 zunächst das durch den Strahlteiler 45 und die Spiegel 41 und 42 gebildete Michelson Interferometer durchlaufen. Wird der Absorber 44 aus dem Strahlengang genommen, so entstehen zwei zueinander verzögerte Strahlen, die über die Arme 1 und 2 auf das Objektiv 10 und das Auge 9 geleitet werden. Aus Gründen der Übersichtlichkeit ist der am Spiegel 42 reflektierte Strahl nicht eingezeichnet. Ist das Licht der Quelle 6 kurzkohärent, so entsteht ein Interferenzbild aus Referenzlichtwelle 29 und Objektlichtwelle 30 nur dann, wenn der Abstand zwischen der Hornhaut und der Spiegelfläche 36 bis auf die Kohärenzlänge gleich der Differenz der Längen der durch die Spiegel 41 und 42 gebildeten Interferometerarme ist. Das Auftreten des Interferenzbildes kann automatisch detektiert werden, indem der Spiegel 42 entlang des Doppelpfeils verschoben wird. Der an dem bewegten Spiegel reflektierte Strahl erfährt dann eine Dopplerverschiebung, was zu einer Schwebung des Interferenzbildes mit der Dopplerfrequenz führt. Diese Schwebung wird durch ein Wechselsignal der Photodiode 26 angezeigt. Wird die zugehörige Position des Spiegels 42 registriert, so kann der Abstand der Hornhaut von der Referenzfläche 36 bis auf die Kohärenzlänge bestimmt werden.

Die Auswertung der Interferenzmuster auf der Fläche 13 der Matrix-CCD-Sensoren kann mit Hilfe eines Rechners 39 erfolgen. Bei der Vorrichtung der Erfindung kann das Auge mit Hilfe der Linsensysteme 11 und 12 in die Detektorebene 13 abgebildet werden. Daher kann das auf dem CCD-Sensor entstehende Bild des Auges auf einem Bildschirm 40 angezeigt werden und damit zur Justierung der Apparatur dienen.

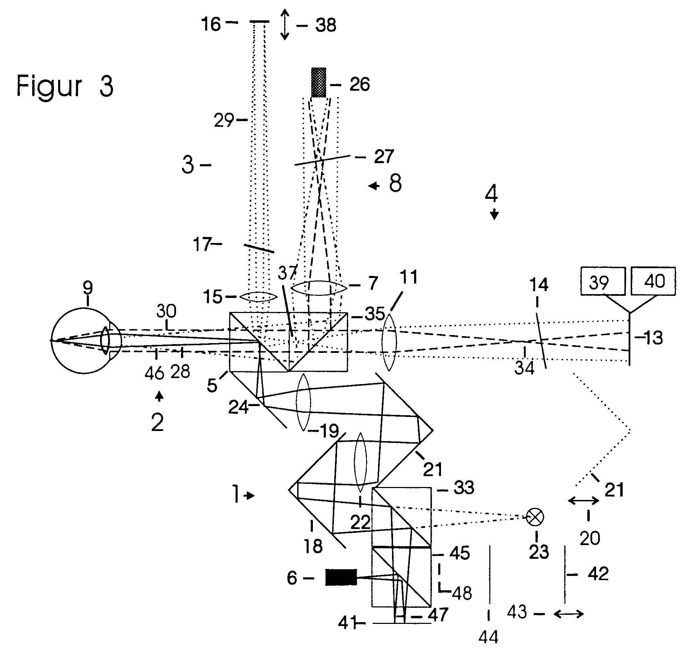

Die Strahlführung ist bei der Vorrichtung der Fig. 3 analog zu der für Fig. 1 geschilderten. Das Licht der Laserdiode 6 wird auf den Strahlteiler 5 geleitet und dort in die ausgezogen gezeichnete Objektlichtwelle 28 und die gepunktet gezeichnete Referenzlichtwelle 29 aufgeteilt. Soll der Augenhintergrund tomographiert werden, wird zunächst das Prisma 21 so verschoben, daß die Punktlichtquelle scharf auf den Augenhintergrund abgebildet wird. Dies kann wie im Zusammenhang mit dem Refraktometer geschildert automatisch durch die Interferenz aus der Überlagerung der Referenzstrahlen 29 und der Objektstrahlen 30 von der Photodiode 26 detektiert werden. Dann wird die Lichtquelle 6 durch eine kurzkohärent emitie rende Quelle ersetzt und nach Entfernen des Absorbers 44 durch das mit dem Strahlteiler 45 und den Spiegeln 41 und 42 gebildete Michelson-Interferometer zwei zueinander verzögerte Strahlen erzeugt. Der auf den Spiegel 42 fallende Strahl ist aus Gründen der Übersichtlichkeit nicht eingezeichnet. Analog zu der für Fig. 2 geschilderten Abstandsmessung kann dann durch Verschieben des Spiegels 42 in Richtung des Doppelpfeils 43 auch hier der Abstand zweier Reflektoren, z.B. Hornhaut und Augenhintergrund detektiert werden. Dazu werden die betreffenden Strahlenbündel - in dem genannten Beispiel Augenhintergrundreflex 30 und Hornhautreflex 46 - in der Ebene des Detektors 26 zur Überlagerung gebracht. Wird nicht nur das Wechselsignal der Photodiode selber, sondern auch dessen Modulationtiefe gemessen, so lassen sich tomographische Messungen machen. Durch Schwenken der Apparatur um den Knotenpunkt des Auges 9 lassen sich verschiedene Punkte des Augenhintergrundes abtasten.

Zur Erhöhung des Kontrastes des Interferenzbildes kann mit Hilfe der Blenden 47 und 48, die durch die Linsen 19 und 22 optisch konjugiert zur Hornhaut liegen, eine Pupillenteilung erreicht werden, so daß eine Reduzierung der inkohärenten Beiträge erfolgt, die den Kontrast herabsetzen würden. Werden sie außerdem mit Filtern kombiniert, so läßt sich die Bestrahlungsstärke der interferierenden Strahlen in der Detektorebene anpassen, was auch eine Kontrastanhebung nach sich zieht. Ebenfalls im Interesse eines guten Kontrastes des Interferenzbildes kann unerwünschtes Streulicht mit Hilfe der Polarisationsfilter 14 bzw. 27 reduziert werden.

Durch geeignete Wahl der Position des Prismas 21 und nötigenfalls durch zusätzliches Einfügen von Linsen in den optischen Arm 1 kann das Strahlbündel 28 so auf das Auge gelenkt werden, daß andere Reflexe als die vom Augenhintergrund, z.B. von der Rückseite der Hornhaut mit dem Hornhautreflex in der Ebene der Photodiode 26 zur Überlagerung kommen. Auf diese Weise kann das gesamte Auge tomographiert werden.

Bei der Vorrichtung der Erfindung kann das Auge mit Hilfe des Linsensystems 11 in die Detektorebene 13 abgebildet werden. Daher kann das auf dem CCD-Sensor entstehende Bild des Auges auf einem Bildschirm 40 angezeigt werden und damit zur Justierung der Apparatur dienen.

**Patentansprüche**

1. Verfahren zum Untersuchen des Auges mit einer Lichtquelle und einem Interferometer, bei dem in einem ersten Strahlengang das Auge angeordnet wird und bei dem die von einer Grenzfläche des Auges zurückgeworfene Objektlichtwelle mit einer Referenzlichtwelle zur Interferenz gebracht wird und die Interferenzerscheinung ausgewertet wird, dadurch gekennzeichnet, daß die Detektion der Interferenz mittels mindestens eines opto-elektronischen Sensors erfolgt und die Referenzlichtwelle durch definierte optische Elemente zur Interferenz mit der Objektlichtwelle geführt wird.

2. Vorrichtung zum Untersuchen des Auges mit einer Lichtquelle und einem Interferometer mit einem Interferometerarm, in dessen Strahlengang das zu untersuchende Auge bringbar ist, mit Einrichtungen zur Detektion und Auswertung der Interferenz zwischen der von einer Grenzfläche des Auges zurückgeworfene Objektlichtwelle mit einer Referenzlichtwelle, dadurch gekennzeichnet, daß die Detektionseinrichtungen mindestens einen opto-elektronischen Sensor (13,26) aufweisen und die Referenzlichtwelle durch definierte optische Elemente zur Interferenz mit der Objektlichtwelle geführt wird.

3. Vorrichtung nach Anspruch 2, dadurch gekennzeichnet, daß vor der Detektorebene eine Abbildungsoptik (11) angeordnet ist.

4. Vorrichtung nach Anspruch 2 oder 3, dadurch gekennzeichnet, daß in der Detektorebene ein CCD-Sensor (13) angeordnet ist.

5. Vorrichtung nach einem der Ansprüche 2 bis 4, dadurch gekennzeichnet, daß der CCD-Sensor (13) das Licht des Interferogramms nur während eines kurzen Zeitraums aufintegriert.

6. Vorrichtung nach einem der Ansprüche 2 bis 5, dadurch gekennzeichnet, daß in den ersten optischen Arm eine Testmarke (23) einspiegelbar ist, deren scheinbare Entfernung vom Auge durch Verschieben einer Spiegelfläche (21) veränderbar ist.

7. Vorrichtung nach einem der Ansprüche 2 bis 6, dadurch gekennzeichnet, daß die kohärente Lichtquelle (6) eine Punktlichtquelle ist und ihre scheinbare Entfernung vom Auge durch Verschieben einer Spiegelfläche (21) veränderbar ist.

8. Vorrichtung nach einem der Ansprüche 2 bis 7, dadurch gekennzeichnet, daß in einem optischen Arm ein fotoempfindliches Element, insbesondere eine Fotodiode (26) angeordnet ist, und daß elektronische Schaltungen zur Feststellung einer Schwebungsfrequenz des Interferenzbildes vorgesehen sind.

9. Vorrichtung nach einem der Ansprüche 2 bis 8, dadurch gekennzeichnet, daß die Lichtquelle (6) ein Laser, insbesondere ein Infrarotlaser ist.

10. Vorrichtung nach einem der Ansprüche 2 bis 9, dadurch gekennzeichnet, daß in den Interferometerarm, in den das Auge (9) bringbar ist, ein Objektiv (10) einbringbar ist.

11. Vorrichtung nach einem der Ansprüche 2 bis 10, dadurch gekennzeichnet, daß der Referenzstrahl in einem separaten Interferometerarm, der an seinem Ende einen Spiegel (16) trägt, erzeugbar ist.

12. Vorrichtung nach Anspruch 11, dadurch gekennzeichnet, daß der Spiegel (16) senkrecht zur Strahlrichtung hin- und herbewegbar ist.

13. Vorrichtung nach einem der Ansprüche 2 bis 12, dadurch gekennzeichnet, daß sie eine oder mehrere Lichtquellen (6) aufweist, die Licht mit unterschiedlichen Wellenlängen emitieren.

14. Vorrichtung nach einem der Ansprüche 2 bis 13, dadurch gekennzeichnet, daß sie zur Justierung eine Foto- oder Quadrantendiode aufweist.

15. Vorrichtung nach einem der Ansprüche 2 bis 14, dadurch gekennzeichnet, daß die Kohärenzlänge der Lichtquelle (6) zeitlich veränderbar ist.

16. Vorrichtung nach einem der Ansprüche 2 bis 14, dadurch gekennzeichnet, daß die Lichtquelle (6) Licht kurzer Kohärenzlänge emitiert.

17. Vorrichtung nach einem der Ansprüche 2 bis 16, dadurch gekennzeichnet, daß die Strahlung der Lichtquelle (6) in zwei zueinander verzögerte Wellen aufgeteilt ist.

Figur 1

Figur 2

EP 0 563 454 A1

Figur 3

EP 0 563 454 A1

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5 ) |
|---|---|---|---|
| X<br>Y | DE-A-3 612 787 (E.ROTH)<br>* das ganze Dokument *<br><br>--- | 1-4,9<br>6,7,<br>10-13,<br>15,16 | A61B3/10<br>A61B3/103<br>A61B3/107<br>G01B11/24 |
| Y | US-A-4 755 041 (Y.ISHIKAWA ET AL.)<br>* das ganze Dokument *<br>--- | 6,16 | |
| Y | EP-A-0 447 067 (INTELLIGENT SURGICAL LASERS INC.)<br>* das ganze Dokument *<br>--- | 10 | |
| D,Y | DE-A-3 201 801 (A.F.FERCHER)<br>* das ganze Dokument *<br>--- | 7,11,12 | |
| Y | EP-A-0 348 057 (KOWA CO. LTD.)<br>* das ganze Dokument *<br>--- | 13,15 | |
| P,X | DE-A-4 037 798 (H.KASPRZAK ET AL.)<br>* das ganze Dokument *<br>--- | 1,2,17 | |
| A | US-A-4 019 813 (T.N.CORNSWEET ET AL.)<br>* Zusammenfassung; Abbildung 1 *<br>--- | 1,2 | RECHERCHIERTE SACHGEBIETE (Int. Cl.5 ) |
| A | TECHNISCHES MESSEN TM<br>Bd. 58, Nr. 6, Juni 1991, MUNCHEN DE<br>Seiten 228 - 234 , XP000235778<br>H.J.TIZIANI 'Kohärent-optische Verfahren in der Oberflächenmesstechnik'<br>* das ganze Dokument *<br><br>----- | 1,10 | A61B<br>G01B |

Der vorliegende Recherchenbericht wurde für alle Patentansprüche erstellt

| Recherchenort | Abschlußdatum der Recherche | Prüfer |
|---|---|---|
| DEN HAAG | 16 JULI 1993 | HUNT B.W. |

KATEGORIE DER GENANNTEN DOKUMENTE

X : von besonderer Bedeutung allein betrachtet
Y : von besonderer Bedeutung in Verbindung mit einer
    anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder
    nach dem Anmeldedatum veröffentlicht worden ist
D : in der Anmeldung angeführtes Dokument
L : aus andern Gründen angeführtes Dokument
........................................................................
& : Mitglied der gleichen Patentfamilie, übereinstimmendes
    Dokument

EPO FORM 1503 03.82 (P0403)